(19) Europäisches Patentamt
European Patent Office
Office européen des brevets

(11) **EP 2 818 154 A1**

(12) **EUROPEAN PATENT APPLICATION**
published in accordance with Art. 153(4) EPC

(43) Date of publication:
**31.12.2014 Bulletin 2015/01**

(21) Application number: **13751099.6**

(22) Date of filing: **22.02.2013**

(51) Int Cl.:
*A61K 8/31* (2006.01)    *A61K 8/35* (2006.01)
*A61K 8/37* (2006.01)    *A61K 8/55* (2006.01)
*A61K 8/60* (2006.01)    *A61K 8/67* (2006.01)
*A61K 31/01* (2006.01)    *A61K 31/122* (2006.01)
*A61K 47/24* (2006.01)    *A61K 47/26* (2006.01)
*A61K 47/34* (2006.01)    *A61K 47/36* (2006.01)
*A61P 17/00* (2006.01)    *A61P 43/00* (2006.01)
*A61Q 1/14* (2006.01)    *A61Q 19/00* (2006.01)
*A61Q 19/08* (2006.01)    *A61Q 19/10* (2006.01)

(86) International application number:
**PCT/JP2013/054611**

(87) International publication number:
**WO 2013/125708 (29.08.2013 Gazette 2013/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **24.02.2012 JP 2012039374**

(71) Applicant: **FUJIFILM Corporation
Tokyo 106-8620 (JP)**

(72) Inventors:
• **INUI, Eriko
  Ashigarakami-gun
  Kanagawa 258-8577 (JP)**
• **KANAZAWA, Katsuhiko
  Ashigarakami-gun
  Kanagawa 258-8577 (JP)**

(74) Representative: **Morpeth, Fraser Forrest
Fujifilm Imaging Colorants Limited
Intellectual Property Group
P.O. Box 42
Hexagon Tower
Blackley, Manchester M9 8ZS (GB)**

(54) **TOPICAL SKIN PREPARATION AND HEALTHY SKIN CELL ACTIVATION AGENT**

(57) A topical skin preparation and a normal skin cell activating agent that include lycopene and astaxanthin, a total content of the lycopene and the astaxanthin is in a range of from 0.00001% by mass to 0.2% by mass.

EP 2 818 154 A1

**Description**

Technical Field

The present invention relates to a topical skin preparation and a normal skin cell activation agent.

Background Art

Carotenoids are known to have a variety of physiological activities. For example, in Mol. Cancer Ther., 2005, Vol. 4(1), pp. 177-186, the relationship between certain kinds of cancers and carotenoids is disclosed. Furthermore, it is disclosed in Japanese National-Phase Publication (JP-A) No. 2009-511570 that lycopene or a derivative thereof suppresses the propagation of breast cancer cells or prostate cancer cells. However, a detailed investigation on the effect on other cells has not been conducted.

In the field of cosmetics, cosmetic products obtained by incorporating lycopene, which is a kind of carotenoid, are known. It is described that when lycopene is incorporated into a cosmetic product, effects of antioxidation, active oxygen removal, and the like can be obtained (for example, Japanese Patent Application Laid-Open (JP-A) No. 2000-229827 and JP-A No. 2002-128651).

It is also known that primrose, lycopodium, and the like have an effect of activating dermal fibroblast cells or epidermal cells (Japanese Patent No. 3507055 and Japanese Patent No. 3389580).

SUMMARY OF INVENTION

Technical Problem

As described above, the physiological activity of lycopene has been hitherto studied, but the overall circumstances thereof have not yet been elucidated.

On the other hand, when a component having a physiologically activating effect such as cell activation is incorporated into a preparation such as a topical skin preparation, stability over time of the preparation is also required. However, a topical skin preparation which simultaneously realizes an excellent cell activating effect and favorable stability over time is has not yet been provided.

The invention has been achieved under such circumstances, and an object of the invention is to provide a topical skin preparation having a cell activating effect and excellent stability over time.

Solution to Problem

The means for solving the problems described above are as follows.

[1] A topical skin preparation, containing: lycopene and astaxanthin, a total content of the lycopene and the astaxanthin is in a range of from 0.00001% by mass to 0.2% by mass.

[2] The topical skin preparation as described in [1], wherein the lycopene is incorporated as a dispersion containing lycopene.

[3] The topical skin preparation as described in [2], including the dispersion that contains the lycopene; at least one selected from a fatty acid ester of a monosaccharide, a fatty acid ester of a polysaccharide, a polyglycerin fatty acid ester of a monosaccharide, or a polyglycerin fatty acid ester of a polysaccharide; and a lecithin.

[4] The topical skin preparation as described in any one of [1] to [3], wherein the astaxanthin is incorporated as a dispersion containing astaxanthin.

[5] The topical skin preparation as described in the item [4], including the dispersion that contains the astaxanthin; at least one selected from a fatty acid ester of a monosaccharide, a fatty acid ester of a polysaccharide, a polyglycerin fatty acid ester of a monosaccharide, or a polyglycerin fatty acid ester of a polysaccharide; and a lecithin.

[6] The topical skin preparation as described in any one of [1] to [5], wherein the mass ratio of the lycopene and the astaxanthin is 0.01 : 1 to 25 : 1.

[7] The topical skin preparation as described in any one of [1] to [6], further including at least one selected from the compound group consisting of tocopherol and derivatives thereof.

[8] The topical skin preparation as described in [7], wherein a mass ratio between the lycopene and the astaxanthin, and the at least one selected from the compound group consisting of tocopherol and derivatives thereof, is from 1 : 50 to 1 : 0.5.

[9] The topical skin preparation as described in any one of [1] to [8], wherein the topical skin preparation is an aqueous cosmetic.

[10] A normal skin cell activation agent, containing: lycopene and astaxanthin, a total content of the lycopene and the astaxanthin is in a range of from 0.00001% by mass to 0.2% by mass.

[11] The normal skin cell activation agent as described [10], wherein a mass ratio of the lycopene and the astaxanthin is from 0.01 : 1 to 25 : 1.

[12] A topical skin preparation including the normal skin cell activation agent as described in [10] or [11].

[13] A cosmetic including the normal skin cell activation agent as described in the item [10] or [11].

Advantageous Effects of Invention

According to the invention, a topical skin preparation having a cell activating effect and an excellent stability over time can be provided.

DESCRIPTION OF EMBODIMENTS

Hereinafter, the present invention will be described in detail.

In the present specification, any numerical range expressed herein using "to" refers to a range including the numerical values before and after "to" as the minimum and maximum values, respectively.

The term "aqueous phase" as used in the invention is used as a term opposing the "oil phase", irrespective of the kind of the solvent.

In the present specification, the term "process" encompasses an independent process, as well as a process that cannot be clearly distinguished from another process but yet achieves the expected effect of the process of interest.

In a case in which the amount of a component in the composition is indicated in the invention, when there are plural substances corresponding to the component in the composition, the indicated amount means the total amount of the plural substances present in the composition, unless specifically stated otherwise.

[Topical skin preparation]

The topical skin preparation of the invention contains lycopene and astaxanthin, a total content thereof is in the range of from 0.00001% by mass to 0.2% by mass.

The topical skin preparation of the invention contains both lycopene and astaxanthin, and when the total content of the compounds is set to a specific range, both the cell activating effect and the stability in the case of being formulated into a preparation can be exhibited.

In the topical skin preparation of the invention, lycopene and astaxanthin are incorporated as components that can function as active ingredients having a cell activating effect in normal skin cells.

Here, the "cell activating effect" in the invention means promoting proliferation or activity enhancement of skin cells, and consequently promoting the effects of anti-wrinkling, anti-aging, anti-sagging, moisturization, and the like in the skin.

Furthermore, the term "stability over time" as used in the invention means that in the case of formulating a topical skin preparation, stability of the preparation is sustained over time without being impaired.

Hereinafter, the various components included in the topical skin preparation of the invention will be described in detail.

In the topical skin preparation of the invention, lycopene and astaxanthin are components that are incorporated as functional components having a cell activating effect in normal skin cells.

In the topical skin preparation of the invention, a total content of lycopene and astaxanthin is in a range of from 0.00001% by mass to 0.2% by mass, and in this range, the cell activating effect can be enhanced, and excellent stability over time is also obtained.

The lower limit of the total content of lycopene and astaxanthin is 0.00001% by mass or more, preferably 0.00005% by mass or more, and more preferably 0.0001% by mass or more. When the total content of lycopene and astaxanthin is less than 0.00001% by mass, a cell activating effect cannot be obtained.

The total content of lycopene and astaxanthin is more preferably from 0.0005% by mass to 0.2% by mass, and still more preferably from 0.001% by mass to 0.1% by mass.

The upper limit of the total content of lycopene and astaxanthin is 0.2% by mass or less, and when the total content is more than 0.2% by mass, stability over time is impaired. For example, in the case of a preparation which is required to have transparency, turbidity increases, and product value is lowered.

The mass ratio of lycopene and astaxanthin (lycopene:astaxanthin) is preferably from 0.01 : 1 to 25 : 1, more preferably from 0.05 : 1 to 15 : 1, and still more preferably 0.05 : 1 to 10 : 1.

<Lycopene>

Lycopene in the invention is a carotenoid represented by chemical formula: $C_{40}H_{56}$, and is a red pigment which belongs to carotenes, one class of carotenoids, and exhibits an absorption maximum at 474 nm (acetone).

Lycopene also includes cis- and trans-isomers, each having a conjugated double bond at the center of the molecule, and examples include an all-trans form, a 9-cis form, and a 13-cis form. In the invention, any of these may be used.

Lycopene may also be incorporated into the topical skin preparation of the invention as a lycopene-containing oil that is isolated or extracted from a natural product containing lycopene, or as a lycopene-containing paste.

Lycopene is contained in tomatoes, persimmons, watermelons, and pink grapefruits in nature, and the lycopene-containing oil may be an oil isolated or extracted from these natural products. Regarding the forms of manufactured products, four kinds such as oil type, emulsion type, paste type, and powder type are known.

Furthermore, the lycopene used in the invention may be an extract, a product obtained by appropriately purifying an extract as necessary, or a synthetic product.

One of particularly preferred forms of lycopene in the invention may be an oil-soluble extract that is extracted from tomato pulp. The oil-soluble extract extracted from tomato pulp is particularly preferable from the viewpoints of stability in a composition including the oil-soluble extract, product quality, or productivity.

Here, an oil-soluble extract extracted from tomato pulp means an extract that is extracted, using an oily solvent, from a pulp-like solid which is obtainable by centrifuging a ground product obtained by grinding tomatoes.

Regarding lycopene as an oil-soluble extract, tomato extracts that are broadly available in the market as lycopene-containing oils or pastes can be used, and examples include LYC-O-MATO 15% and LYC-O-MATO 6% available from Sunbright Co., Ltd.; and LYCOPENE 18 available from Kyowa Hakko Kogyo Co., Ltd.

In the topical skin preparation of the invention, lycopene is preferably incorporated as a dispersion that contains lycopene. When lycopene is incorporated in the form of dispersion, lycopene can be incorporated stably in the system of the topical skin preparation.

The dispersion containing lycopene may be an oil-in-water type dispersion (O/W type dispersion), or may be a water-in-oil type dispersion (W/O type dispersion), and the form can be selected in accordance with the formulation of the topical skin preparation. The dispersion containing lycopene is more preferably an oil-in-water type dispersion containing lycopene as one of oil phase components.

The dispersion containing lycopene may include astaxanthin together with lycopene, so that the dispersion may also serve as a dispersion containing astaxanthin which will be described below.

The content of lycopene in the topical skin preparation of the invention may vary depending on the formulation of the topical skin preparation, and the content is preferably from more than 0.00001% by mass to less than 0.2% by mass, more preferably from 0.00005% by mass to 0.1% by mass, and still more preferably from 0.0001% by mass to 0.05% by mass, with respect to the total mass of the topical skin preparation.

<Astaxanthin>

Astaxanthin according to the invention includes at least one of astaxanthin or derivatives of astaxanthin, such as esters thereof. In the invention, unless particularly stated otherwise, these are collectively referred to as "astaxanthin".

Regarding the astaxanthin used in the invention, any of astaxanthin obtainable from naturally occurring products such as plants, algae, crustaceans, or bacteria, as well as products obtainable according to conventional methods, can be used.

Examples of astaxanthin as a natural product include red Phaffia yeast, green alga Haematococcus, marine bacteria, and krill. Further examples include extracts obtained from extracts of the cultures of those organisms.

Astaxanthin may be included in the topical skin preparation of the invention as an astaxanthin-containing oil, which is obtainable as an isolation product or an extract from natural products containing astaxanthin. Astaxanthin may be a product obtained by appropriately further purifying, if necessary, an isolation product or an extract from these natural products, or may also be a synthetic product.

For astaxanthin, a product extracted from Haematococcus alga (also referred to as a Haematococcus alga extract), and a krill-derived pigment are particularly preferred from the viewpoint of product quality or productivity.

Specific examples of astaxanthin derived from a Haematococcus alga extract that can be used in the invention include astaxanthin derived from extracts obtained from Haematococcus pluvialis, Haematococcus lacustris, Haematococcus capensis, Haematococcus droebakensis, and Haematococcus zimbabwiensis, but the invention is not intended to be limited to these.

The Haematococcus alga extract that can be used in the invention may also be obtained by crushing the cell walls of the raw materials described above, if necessary, for example, by the method disclosed in JP-A No. H05-68585 or the like; adding thereto an organic solvent such as acetone, ether, chloroform, or an alcohol (ethanol, methanol, or the like), or an extracting solvent such as carbon dioxide in a supercritical state; and performing extraction.

Furthermore, in this invention, Haematococcus alga extracts that are broadly available in the market can be used, and examples of commercially available extracts include ASTOTS-S, ASTOTS-2.5 O, ASTOTS-5 O, and ASTOTS-10 O manufactured by Takedashiki Co., Ltd.; ASTAREAL OIL 50F and ASTAREAL OIL 5F manufactured by Fuji Chemical Industry Co., Ltd.; and BIOASTIN SCE7 manufactured by Toyo Koso Kagaku Co., Ltd.

The content of astaxanthins as a pure pigment component in the Haematococcus alga extract that can be used in the invention is preferably from 0.001% by mass to 50% by mass, and more preferably from 0.01% by mass to 25% by mass, from the viewpoint of handling at the time of producing a composition.

The Haematococcus alga extract that can be used in the invention may include astaxanthin or an ester form thereof as a pure pigment component as in the case of the pigment described in JP-ANo. H02-49091, and in that case, a Haematococcus alga extract containing an ester form in an amount of generally 50 mol% or more, preferably 75 mol% or more, and more preferably 90 mol% or more, can be preferably used.

In the topical skin preparation of the invention, astaxanthin is preferably incorporated as a dispersion containing astaxanthin. When astaxanthin is incorporated in the form of dispersion, astaxanthin can be incorporated stably into the system of the topical skin preparation.

The dispersion containing astaxanthin may be an oil-in-water type dispersion (O/W type dispersion), or may be a water-in-oil type dispersion (W/O type dispersion), and can be selected in accordance with the formulation of the topical skin preparation. The dispersion containing astaxanthin is more preferably an oil-in-water type dispersion containing astaxanthin as one of oil phase components.

The content of astaxanthin in the topical skin preparation of the invention can be appropriately selected depending on the form of the topical skin preparation, as long as the requirement with respect to the total content of lycopene and astaxanthin according to the invention is met. From the viewpoints of a cell activating effect and stability over time, the content is preferably from more than 0.00001% by mass to less than 0.2% by mass, more preferably from 0.00005% by mass to 0.1 % by mass, and still more preferably from 0.0001 % by mass to 0.05% by mass, with respect to the total mass of the topical skin preparation.

<Surfactant>

When the topical skin preparation of the invention includes lycopene in the form of a dispersion containing lycopene, the dispersion containing lycopene is preferably a dispersion containing one kind or two or more kinds of surfactants. Furthermore, in regard to the topical skin preparation of the invention, when the formulation of the topical skin preparation is configured as an emulsified composition such as an emulsion, the topical skin preparation may contain a surfactant as an emulsifying agent.

Furthermore, when the topical skin preparation of the invention includes astaxanthin in the form of a dispersion containing astaxanthin, the dispersion containing astaxanthin is preferably a dispersion containing one kind or two or more kinds of surfactants.

Also, in regard to the topical skin preparation of the invention, when the formulation of the topical skin preparation is configured as an emulsified composition such as an emulsion, the topical skin preparation may contain a surfactant as an emulsifying agent.

The surfactant that can be applied to the invention may be any one of an anionic surfactant, a cationic surfactant, an amphoteric surfactant, and a nonionic surfactant.

Furthermore, from the viewpoint of emulsifying power, the surfactant according to the invention preferably has an HLB value of 10 or more, and more preferably 12 or more. When the HLB value is too low, the emulsifying power may be insufficient. In addition, from the viewpoint of a defoaming effect, a surfactant having an HLB value of from 5 to less than 10 may also be used together.

Here, HLB stands for hydrophilicity-lipophilicity balance that is ordinarily used in the field of surfactants, and an ordinarily used calculation formula, for example, Kawakami's formula, can be used. Kawakami's formula is presented below.

$$HLB = 7 + 11.7 \log (Mw/Mo)$$

Here, Mw represents the molecular weight of a hydrophilic group, and Mo represents the molecular weight of a hydrophobic group.

Furthermore, the values of HLB described in catalogues and the like may also be used.

Furthermore, as can be seen from the above formula, an emulsifying agent having an arbitrary HLB value can be obtained by utilizing the additive properties of HLB.

Among the surfactants, a nonionic surfactant is preferred in view of being less irritating and having less influence on the environment. Examples of the nonionic surfactant include a fatty acid ester of a monosaccharide or a polysaccharide, a polyglycerin fatty acid ester, an organic acid monoglyceride, a propylene glycol fatty acid ester, a polyglycerin-condensed ricinoleic acid ester, a sorbitan fatty acid ester, and a polyoxyethylene sorbitan fatty acid ester.

Regarding the surfactants included in the dispersion containing lycopene and the dispersion containing astaxanthin, from the viewpoint of stability of the dispersions, it is preferable that the surfactants include at least one selected from a fatty acid ester of a monosaccharide or a polysaccharide, or a polyglycerin fatty acid ester.

Examples of the monosaccharide or polysaccharide that constitutes the fatty acid ester of a monosaccharide or a polysaccharide include glucose and sucrose.

The fatty acid ester of a monosaccharide or a polysaccharide is more preferably an ester form of a monosaccharide or a polysaccharide and a linear or branched fatty acid having 6 to 22 carbon atoms. From the viewpoint of producing a topical skin preparation having high stability, sucrose myristic acid ester, sucrose palmitic acid ester, sucrose stearic acid ester, and sucrose oleic acid ester are still more preferred, and sucrose stearic acid ester is most preferred.

The polyglycerin fatty acid ester is more preferably an ester form between a polyglycerin having a degree of polymerization of 2 or more and a linear or branched fatty acid having 6 to 22 carbon atoms.

From the viewpoint of producing a highly stable topical skin preparation, examples of the polyglycerin fatty acid ester include polyglyceryl-6 laurate, polyglyceryl-6 myristate, polyglyceryl-6 stearate, polyglyceryl-6 oleate, polyglyceryl-10 laurate, polyglyceryl-10 myristate, polyglyceryl-10 stearate, decaglyceryl monooleate, and polyglyceryl-10 oleate are still more preferred. Also, decaglyceryl monooleate and decaglyceryl-10 oleate are most preferred.

(Phospholipid)

In addition, in regard to the topical skin preparation of the invention, in a case in which lycopene is incorporated in the form of a dispersion containing lycopene, the dispersion containing lycopene is preferably a dispersion containing a phospholipid such as a lecithin.

In regard to the topical skin preparation of the invention, in a case in which astaxanthin is incorporated in the form of a dispersion containing astaxanthin, the dispersion containing astaxanthin is preferably a dispersion containing a phospholipid such as a lecithin.

Furthermore, in regard to the topical skin preparation of the invention, when the formulation of the topical skin preparation is configured as an emulsified composition such as an emulsion, a phospholipid may be incorporated as an emulsifying agent.

The phospholipid that can be used in the invention is a compound containing a glycerin skeleton, a fatty acid residue, and a phosphoric acid residue, and having a base, a polyhydric alcohol, or the like bonded thereto, and the phospholipid is also referred to as lecithin. Since a phospholipid has a hydrophilic group and a hydrophobic group in the molecule, phospholipids have been widely used hitherto in the fields of foods, pharmaceuticals and cosmetics as an emulsifying agent.

From an industrial viewpoint, lecithin having a purity of 60% or higher is utilized as lecithin, and this can also be utilized in this invention. However, from the viewpoints of forming a fine oil droplet particle size and having stability of functional oily components, a product that is generally called high purity lecithin is preferred, and this has a lecithin purity of 80% or higher, and more preferably 90% or higher.

Examples of the phospholipid include various conventionally known phospholipids that have been extracted and isolated from the living bodies of plants, animals, and microorganisms.

Specific examples of such a phospholipid include various lecithins derived from plants such as soybean, corn, peanut, rapeseed, or barley; animals such as egg yolk or beef; and microorganisms such as Escherichia coli. Among these, egg yolk-derived lecithin or soybean-derived lecithin is preferred, and soybean-derived lecithin is more preferred.

Examples of such lecithins include, as described in compound names, glycerolecithins such as phosphatidic acid, phosphatidylglycerin, phosphatidylinositol, phosphatidylethanolamine, phosphatidylmethylethanolamine, phosphatidylcholine, phosphatidylserine, bisphosphatidic acid, diphosphatidylglycerin (cardiolipin), phosphatidylethanolamine, phosphatidylinositol, and lysophosphatidylcholine; and sphingolecithins such as sphingomyelin.

Furthermore, in this invention, hydrogenated lecithin, enzymatically decomposed lecithin, enzymatically decomposed hydrogenated lecithin, hydroxylecithin, and the like can be used, in addition to the high purity lecithin described above. Such a lecithin that can be used in the invention can be used singly, or in the form of a mixture of plural kinds.

<Other components>

In the topical skin preparation of the invention, components other than lycopene and astaxanthin can be appropriately selected and incorporated in accordance with the form, purpose, or the like of the topical skin preparation. Suitable examples of the other components include a functional oily component, an emulsifying agent, and other additive components.

(Functional oily component)

The functional oily component is not particularly limited as long as the component is an oil-soluble component which does not dissolve in an aqueous medium but dissolves in an oily medium, and any oily component having the properties or functionality conforming to the purpose can be appropriately selected and used. Examples of other oily components include those oily components which are usually used as an ultraviolet absorber, an anti-inflammatory agent, a moisturizing agent, a hair protective agent, a whitening agent, an anti-blemish agent, a cell activating agent, an emollient, a keratolytic agent, an antistatic agent, an oil-soluble vitamin, a metabolic syndrome improving agent, a hypotensive agent, a sedative agent, and the like.

Here, a functional oily component means an oily component that is expected to exhibit, when present in a living organism, a desired physiological action directed to the living organism.

Examples of the functional oily component include naturally occurring ceramides; ceramides including ceramide and ceramide analogues, such as ceramides modified with sugars such as sphingoglycolipids; oils and fats such as olive oil, camellia oil, macademia nut oil, castor oil, and coconut oil; hydrocarbons such as liquid paraffin, paraffin, petrolatum, ceresin, microcrystalline wax, and squalane; waxes such as carnauba wax, candelilla wax, jojoba oil, beeswax, and lanolin; esters such as isopropyl myristate, 2-octyldodecyl myristate, cetyl 2-ethylhexanoate, and diisostearyl malate; fatty acids such as palmitic acid, stearic acid, and isostearic acid; higher alcohols such as cetyl alcohol, stearyl alcohol, isostearyl alcohol, and 2-octyldodecanol; silicone oils such as methylpolysiloxane and methylphenylpolysiloxane; fatty acid esters of glycerin; fat-soluble vitamins such as vitamin E (tocopherol) family, vitamin A family, and vitamin D family; as well as polymers, oil-soluble pigments such as carotenoids other than lycopene and xanthopyll; and oil-soluble proteins. Furthermore, various plant-derived oils and animal-derived oils, which are mixtures of those components, are also included. In the topical skin preparation of the invention, it is preferable to incorporate vitamin E (tocopherol) family from the viewpoint of markedly enhancing the stability over time of the topical skin preparation containing lycopene and astaxanthin as functional oily components.

Examples of the vitamin E (tocopherol) family include compounds selected from the compound group consisting of tocopherol and derivatives thereof. These may be used singly, or may be used in combination of plural kinds thereof.

The compound group consisting of tocopherol and derivatives thereof includes dl-$\alpha$-tocopherol, dl-$\beta$-tocopherol, dl-$\gamma$-tocopherol, dl-$\delta$-tocopherol, dl-$\alpha$-tocopherol acetate, dl-$\beta$-tocopherol acetate, dl-$\gamma$-tocopherol acetate, dl-$\delta$-tocopherol acetate, dl-$\alpha$-tocopherol nicotinate, dl-$\beta$-tocopherol nicotinate, dl-$\gamma$-tocopherol nicotinate, dl-$\delta$-tocopherol nicotinate, dl-$\alpha$-tocopherol linolate, dl-$\beta$-tocopherol linolate, dl-$\gamma$-tocopherol linolate, dl-$\delta$-tocopherol linolate, dl-$\alpha$-tocopherol succinate, dl-$\beta$-tocopherol succinate, dl-$\gamma$-tocopherol succinate, and dl-$\delta$-tocopherol succinate. Among these, dl-$\alpha$-tocopherol, dl-$\beta$-tocopherol, dl-$\gamma$-tocopherol, dl-$\delta$-tocopherol, and mixtures thereof (mixed tocopherols) are more preferred. Furthermore, preferred examples of tocopherol derivatives include dl-$\alpha$-tocopherol acetate, dl-$\beta$-tocopherol acetate, dl-$\gamma$-tocopherol acetate, dl-$\delta$-tocopherol acetate, and mixtures thereof (tocopherol acetate).

The content of tocopherol and derivatives thereof in the topical skin preparation of the invention is preferably from 0.00001% by mass to 2% by mass, more preferably from 0.00005% by mass to 1.5% by mass, and still more preferably from 0.0001% by mass to 1% by mass, with respect to the total mass of the topical skin preparation.

The mass ratio between lycopene and astaxanthin, and tocopherol and derivatives thereof (lycopene + astaxanthin : tocopherol and derivatives thereof) is preferably from 1 : 50 to 1 : 0.5, more preferably from 1 : 25 to 1 : 0.75, and still more preferably from 1 : 10 to 1 : 1.

(Other additive components)

In addition to the components described above, additive components that are ordinarily used in the fields of foods, cosmetics, and the like may be incorporated, if appropriate, into the topical skin preparation of the invention according to the form of the preparation.

Other additive components can be incorporated into the topical skin preparation of the invention as oil-soluble or water-soluble additive components, depending on the characteristics thereof.

For example, examples of the other additive components include polyhydric alcohols such as glycerin and 1,3-butylene glycol; monosaccharides or polysaccharides, such as kappa-carrageenan, locust bean gum, guar gum, hydroxypropyl guar gum, xanthan gum, karaya gum, tamarind seed polysaccharides, gum arabic, tragacanth gum, hyaluronic acid, sodium hyaluronate, sodium chondroitin sulfate, and dextrin; sugar alcohols such as sorbitol, mannitol, maltitol, lactose, maltotriitol, and xylitol; inorganic salts such as sodium chlorideand sodium sulfate; proteins having molecular weights of more than 5000, such as casein, albumin, methylated collagen, hydrolyzed collagen, water-soluble collagen, and gelatin; amino acids such as glycine, alanine, valine, leucine, isoleucine, serine, threonine, aspartic acid, glutamic acid, cysteine, methionine, lysine, hydroxylysine, arginine, histidine, phenylalanine, tyrosine, tryptophan, proline, hydroxyproline, and acetylhydroxyproline, and derivatives thereof; synthetic polymers such as carboxyvinyl polymers, sodium polyacrylate, polyvinyl alcohol, polyethylene glycol, and ethylene oxide-propylene oxide block copolymers; water-soluble cellulose derivatives such as hydroxyethyl cellulose and methyl cellulose; flavonoids (catechin, anthocyanin, flavone, isoflavone, flavane, flavanone, and rutin), ascorbic acid, or derivatives thereof (ascorbic acid, sodium ascorbate, potassium ascorbate, calcium ascorbate, L-ascorbic acid phosphoric acid ester, magnesium ascorbyl phosphate, sodium ascorbyl phosphate, ascorbyl sulfate, ascorbyl sulfate disodium salt, and ascorbyl-2-glucoside, and the like), tocotrienol and derivatives thereof, phenolic acids (chlorogenic acid, ellagic acid, gallic acid, and propyl gallate), lignans, curcumins, coumarins, and hydroxystilbene including pterostilbene and the like. The additive components may also include, for example, a functional component, an excipient, a viscosity adjusting agent, and a radical scavenger, based on the function.

In addition, in the invention, for example, other additives that are ordinarily used for relevant uses, such as various efficacious components, a pH adjusting agent, a pH buffering agent, an ultraviolet absorber, an antiseptic agent, a fragrance, and a colorant, can be used in combination.

There are no particular limitations on the form of the topical skin preparation of the invention, and the topical skin preparation may be in the form of a composition including active ingredients, as well as a pharmaceutically acceptable carrier, and other optional components, if necessary. Examples of the form of the composition include an oil composition and an emulsified composition. The emulsified composition is preferably an oil-in-water type emulsified composition.

<Applications>

The topical skin preparation of the invention can be applied to applications such as application to cosmetics and application to transdermal pharmaceutical products.

Examples of the cosmetic products to which the topical skin preparation of the invention is applied include, but are not limited to, skin-care cosmetic products (skin toner, beauty essence, milky lotion, cream, and the like), lipsticks, ultraviolet screening cosmetics, and makeup cosmetics.

The topical skin preparation of the invention is preferably used particularly in aqueous cosmetic materials such as skin toner, essence, milky lotion, cream mask pack, pack, hair washing cosmetic products, fragrance cosmetic products, liquid body washing materials, UV care cosmetic products, deodorant cosmetic products, and oral care cosmetic products (in the case of cosmetic products). When the topical skin preparation is used in an aqueous cosmetic material, a product having a feeling of transparency is obtained, and the occurrence of any inconvenient phenomenon such as precipitation, sedimentation, or neck ring of insoluble materials in long-term storage or under severe conditions such as a sterilization treatment can be suppressed.

The skin toner and essence are generally required to have transparency, and it is required that the skin toner and essence remain transparent even after the change over time. "Transparency" as used herein means that the absorbance at 625 nm is 0.1 or less, and is an essential performance from the viewpoint of stability of the preparation.

The absorbance that serves as an indicator of "transparency" in the invention can be measured using a known measuring device. For example, the absorbance can be measured using a commercially available spectrophotometer. In this invention, the absorbance is measured using a spectrophotometer (V-630iRM) manufactured by JASCO Corp.

The absorbance described in the present specification is a value measured at room temperature.

<Method of producing topical skin preparation>

The method of producing the topical skin preparation of the invention is not particularly limited, and the topical skin preparation can be produced according to a known method.

For example, in the case of producing a topical skin preparation including at least one of lycopene or astaxanthin as a dispersion, a production method including the processes of a) dissolving a nonionic emulsifying agent in an aqueous medium (water or the like) to obtain an aqueous phase; b) mixing or dissolving lycopene or astaxanthin, or oily components including lycopene and astaxanthin to obtain an oil phase composition; and c) mixing this aqueous phase composition with the oil phase composition under stirring, performing emulsifying dispersion to obtain a dispersion, and then incorporating other components thereto to obtain the topical skin preparation.

Particularly, it is preferable that the oily components such as lycopene and astaxanthin that are included in an oil phase are sufficiently dissolved by performing a heating treatment at a temperature of higher than or equal to the melting point, subsequently the oily components are mixed with an aqueous phase, and an emulsification treatment is carried out.

When both lycopene and astaxanthin are included as dispersions in the topical skin preparation, a dispersion containing lycopene and a dispersion containing astaxanthin may be produced separately, or a dispersion containing both lycopene and astaxanthin may be produced.

In a case in which a topical skin preparation containing at least one of lycopene or astaxanthin as a dispersion is produced by the production method described above, there are no particular limitations on the components that are included in the oil phase composition and the aqueous phase composition. However, from the viewpoint of producing a highly stable topical skin preparation, it is preferable that the topical skin preparation includes components such as a surfactant, and it is preferable that the topical skin preparation contains any one or a plural number of i) a fatty acid ester of a monosaccharide or a polysaccharide; ii) a polyglycerin fatty acid ester; or iii) a phospholipid.

The ratio (mass) between the oil phase and the aqueous phase in the emulsifying dispersion is not particularly limited, but the oil phase/aqueous phase ratio (mass%) is preferably from 0.1/99.9 to 50/50, more preferably from 0.5/99.5 to 30/70, and still more preferably from 1/99 to 20/80.

When the oil phase/aqueous phase ratio is set to 0.1/99.9 or more, since the proportion of the active ingredients is not lowered, there is a tendency that the dispersion containing lycopene or astaxanthin has no problem in practical use, which is preferable. Furthermore, when the oil phase/aqueous phase ratio is set to 50/50 or less, the surfactant concentration is not lowered, and there is a tendency that stability of the dispersion containing lycopene or astaxanthin is not deteriorated, which is preferable.

Regarding the emulsifying dispersion, an emulsification operation of a single step may be carried out, but it is preferable to carry out an emulsification operation of two or more steps from the viewpoint of obtaining uniform and fine particles.

Specifically, it is particularly preferable to use two or more emulsifying apparatuses in combination, through a method of performing emulsifying dispersion using a high pressure homogenizer, an ultrasonic dispersing machine or the like, in addition to a one-step emulsification operation of performing emulsifying dispersion using a conventional emulsifying apparatus that utilizes shear action (for example, a stirrer, an impeller-stirrer, a homomixer, or a continuous flow type shear apparatus). When a high pressure homogenizer is used, the dispersion can be prepared into liquid droplets of more uniform fine particles. Furthermore, the operation may be carried out several times for the purpose of obtaining liquid droplets having a more uniform particle size.

Surface chemistry-based emulsification methods such as a PIT emulsification method and a gel emulsification method are known as useful methods for making the dispersion finer. These methods have an advantage that a smaller amount of energy is consumed, and are thus adequate in the case of emulsifying and dispersing a material that is prone to be deteriorated by heat, to a fine state.

Furthermore, as an emulsification method that is used for general purposes, a method of using a mechanical force, that is, a method of dividing oil droplets by applying a strong shear force from an external source, is applied. The most general example of the mechanical force is a high-speed, high-shear stirring machine. Commercially available examples of such a stirring machine include machines called a Homomixer, a Disper-mixer, and an Ultramixer.

Also, a high pressure homogenizer is available as another mechanical emulsifying apparatus that is useful for micronization, and various apparatuses are commercially available. Since a high pressure homogenizer can provide a large shear force compared with a stirring type machine, micronization is enabled even with a relatively smaller amount of the emulsifying agent.

High pressure homogenizers can be broadly divided into chamber type high pressure homogenizers having a fixed throttle section, and homogenous valve type high pressure homogenizers in which the extent of opening of a throttle is controlled.

Examples of the chamber type high pressure homogenizers include a MICROFLUIDIZER (manufactured by Microfluidics Corp.), a NANOMIZER (manufactured by Yoshida Kikai Co., Ltd.), and an ULTIMIZER (manufactured by Sugino Machine, Ltd.).

Examples of the homogeneous valve type high pressure homogenizer include a Gaulin type homogenizer (manufactured by APV PLC.), a Rannie type homogenizer (manufactured by Rannie Co., Ltd.), a high pressure homogenizer (manufactured by Gea Niro Soavi S.P.A.), a homogenizer (manufactured by Sanwa Machinery Trading Co., Ltd.), a high pressure homogenizer (manufactured by Izumi Food Machinery Co., Ltd.), and an ultrahigh pressure homogenizer (manufactured by Ika Group).

An ultrasonic homogenizer is available as an emulsifying apparatus having a simple structure, which is a dispersing apparatus having relatively high energy efficiency. Examples of a high power output ultrasonic homogenizer capable of production include an ULTRASONIC HOMOGENIZER US-600, an ULTRASONIC HOMOGENIZER US-1200T, an ULTRASONIC HOMOGENIZER RUS-1200T, an ULTRASONIC HOMOGENIZER MUS-1200T (all manufactured by Nissei Corp.); an ULTRASONIC PROCESSER UIP 2000, an ULTRASONIC PROCESSER UIP -4000, an ULTRASONIC PROCESSER UIP-8000, and an ULTRASONIC PROCESSER UIP-16000 (all manufactured by Hielscher Ultrasonics GmbH). These high power output ultrasonic wave irradiating apparatuses are used at a frequency of 25 kHz or less, and preferably from 15 kHz to 20 kHz.

Furthermore, as another known emulsifying means, a method of using a static mixer, a microchannel, a micromixer, a membrane emulsifying apparatus, or the like, each of which does not have an external stirring unit and requires only a small amount of energy, is also a useful method.

There are no particular limitations on the temperature conditions for performing emulsifying dispersion according to the invention, but from the viewpoint of stability of the functional oily components, the temperature is preferably from 10°C to 100°C. A preferred range can be selected as appropriate, based on the melting point of the functional oily component to be handled, or the like.

Furthermore, when a high pressure homogenizer is used in this invention, it is preferable to carry out the treatment at a pressure of preferably 50 MPa or more, more preferably from 50 MPa to 280 MPa, and still more preferably from 100 MPa to 280 MPa.

Furthermore, it is preferable to cool the emulsified liquid, which is an emulsified and dispersed composition, within 30 seconds, and preferably within 3 seconds, immediately after passing through the chamber, using any cooling machine, from the viewpoint of maintaining the particle size of dispersed particles.

[Normal skin cell activating agent]

The normal skin cell activating agent of the invention includes lycopene and astaxanthin, and a total content of lycopene and astaxanthin is in a range of from 0.00001% by mass to 0.2% by mass.

The normal skin cell activating agent of the invention includes both lycopene and astaxanthin, and it has been found that when the total content thereof is adjusted to a specific range, the normal skin cell activating agent exhibits an epidermal keratinocyte proliferation potency in a cell activating effect test using human epidermal keratinocytes.

The normal skin cell activating agent of the invention can be used as a material for topical skin preparations, cosmetics, and transdermal pharmaceuticals.

The normal skin cell activating agent of the invention can be produced in the same manner as for the topical skin preparation described above.

Here, the "normal skin cell" according to the invention refers to an epidermal cell that is not cancerous. Epidermal cells include keratinocytes, melanocytes, Langerhans cells, $\alpha$-dendritic cells, and Merkel cells. The normal skin cells in the invention are preferably epidermal cells including keratinocytes.

The mass ratio between lycopene and astaxanthin (lycopene : astaxanthin) in the normal skin cell activating agent is preferably from 0.01 : 1 to 25 : 1, more preferably from 0.05 : 1 to 15 : 1, and still more preferably from 0.05 : 1 to 10 : 1.

EXAMPLES

Hereinafter, the invention will be described in detail by way of Examples, but the invention is not intended to be limited to the Examples.

[Examples 1-1 to 1-4 and Comparative Examples A-1 to A-2 and B-1 to B-2]

In Examples 1-1 to 1-4 and Comparative Examples A-1 to A-2 and B-1 to B-2, it was confirmed that an excellent cell activating effect is obtained when lycopene and astaxanthin are incorporated in the specific total content according to the invention, as compared with the case of using these compounds singly.

The samples used in Examples 1 to 4 are samples in which the contents of lycopene and astaxanthin were adjusted to be within the range of the total content of these components according to the invention. On the other hand, the samples used in Comparative Examples A-1 to A-2 and B-1 to B-2 are samples in which the contents of lycopene and astaxanthin were adjusted to be outside the range of the total content of these components according to the invention.

<Test for confirming cell activating effect>

A HaCaT cell line (human epidermal keratinocyte cell line, purchased from DKFZ, Germany, and hereinafter, the same) was inoculated on a 96-well plate using 10 v/v% FBS-added DMEM medium, and the cells were cultured at 37°C in 5% $CO_2$ until the cells reached 80% confluence.

For the cell line used in each Example or Comparative Example, a 10 mM solution of lycopene (manufactured by Wako Pure Chemical Industries, Ltd.) dissolved in DMSO, and/or a solution of astaxanthin (manufactured by Wako Pure Chemical Industries, Ltd.) was added such that the final concentration would correspond to the amount of addition indicated in the following Table 1 or Table 2. The medium was replaced with DMEM medium, and the cells were cultured for 24 hours at 37°C in 5% $CO_2$. In addition, a cell line cultured in a medium in which only DMSO was added at a concentration of 0.1% by mass was used as control.

The cultured cells were washed two times with PBS, subsequently the medium was replaced with a solution obtained by dissolving 0.5 mg of MTT reagent (Dojindo) in 1 ml of PBS, and the cells were incubated for 2 hours at 37°C in 5% $CO_2$.

Thereafter, 100 $\mu$l each of a solution of DMSO : PBS = 4 : 1 (v/v) was added to each well, and the plate was subjected to light sonication. The absorbance at 570 nm was measured using a microplate reader (product name:

SUNRISE RAINBOW THERMO RC, manufactured by Tecan Japan Co., Ltd.), and a comparison of the changes in cell proliferation rate (%) in the various samples for evaluation was made, with the control being designated as 100%.

The results of Examples 1-1 to 1-3 and Comparative Examples A-1 to A-2 are presented in the following Table 1. Meanwhile, the amounts of addition of lycopene and astaxanthin are expressed in "mass%", and the values obtained by converting to "μM" are also indicated together in Table 1.

[Table 1]

| | Amount of addition (mass%) | | Lycopene/astaxanthin (mass ratio) | Cell proliferation rate (%) |
|---|---|---|---|---|
| | Lycopene | Astaxanthin | | |
| Control | 0 | 0 | - | 100% |
| Comparative Example A-1 | $5.4 \times 10^{-4}$ (10μM) | 0 | - | 117% |
| Comparative Example A-2 | 0 | $6.0 \times 10^{-4}$ (10μM) | - | 126% |
| Example 1-1 | $2.7 \times 10^{-4}$ (5μM) | $3.0 \times 10^{-4}$ (5μM) | 0.9:1 | 132% |
| Example 1-2 | $1.1 \times 10^{-4}$ (2μM) | $4.8 \times 10^{-4}$ (8μM) | 0.23:1 | 135% |
| Example 1-3 | $5.4 \times 10^{-5}$ (μM) | $5.4 \times 10^{-4}$ (9μM) | 0.1:1 | 150% |

As shown in Table 1, in the samples of the various Examples in which both lycopene and astaxanthin were added, markedly high cell proliferation rates were exhibited, as compared with the samples of the various Comparative Examples in which either one of lycopene or astaxanthin was added. Thus, a synergistic effect in the cell activating effect could be verified.

The results of Example 1-4 and Comparative Examples B-1 to B-2 are presented in the following Table 2. Meanwhile, the amounts of addition of lycopene and astaxanthin are expressed in "mass%", and the values obtained by converting to "μM" are also indicated together in Table 2.

[Table 2]

| | Amount of addition (mass%) | | Lycopene/astaxanthin (mass ratio) | Cell proliferation rate (%) |
|---|---|---|---|---|
| | Lycopene | Astaxanthin | | |
| Control | 0 | 0 | - | 100% |
| Comparative Example B-1 | $5.4 \times 10^{-5}$ (1μM) | 0 | - | 100.3% |
| Comparative Example B-2 | 0 | $6.0 \times 10^{-5}$ (1μM) | - | 107% |
| Example 1-4 | $1.1 \times 10^{-5}$ (0.2 μM) | $4.7 \times 10^{-5}$ (0.8 μM) | 0.23 : 1 | 122% |

Example 1-4 is an example in which the amounts of addition of lycopene and astaxanthin were reduced to about 1/10 of Example 1-2; Comparative Example B-1 is an example in which the amount of addition of lycopene was reduced to 1/10 of the same amount of Comparative Example A-1; and Comparative Example B-2 is an example in which the amount of addition of astaxanthin was reduced to 1/10 of the same amount of Comparative Example A-2.

As shown in Table 2, it was confirmed that even when the amounts of addition of lycopene and astaxanthin were adjusted to 1/10, a superior cell activating effect is obtained by using lycopene and astaxanthin in combination, compared with a case in which these compounds were added singly.

Furthermore, when the results of the cell proliferation rate of Example 1-4 in Table 2 were compared with the results of the cell proliferation rates of Comparative Example A-1 and Comparative Example A-2 in Table 1, the cell activating effect of Example 1-4 was equivalent to the cell activating effect of Comparative Example A-1 and Comparative Example A-2, in which lycopene and astaxanthin were added singly. From these results, it was found that when lycopene and astaxanthin were used together as in the case of Example 1-4, even when the total content was reduced to 1/10, a very high cell activating effect was obtained as compared with a case in which lycopene or astaxanthin was added singly. From this, it can be said that the cell activating effect obtainable by using lycopene and astaxanthin in combination is synergistic.

From the results shown in Table 1 and Table 2, it was confirmed that when lycopene and xanthophyll are used in combination with the specific total content according to the invention, an excellent cell activating effect is obtained. From this, the topical skin preparation of the invention is anticipated to exhibit a superior cell activating effect.

[Examples 2-1 to 2-8 and Comparative Example 2-1]

<Production of astaxanthin dispersion A and lycopene dispersion B>

The astaxanthin dispersion and lycopene dispersion used in the various topical skin preparations of Examples and Comparative Examples were prepared as follows.

«Production of astaxanthin dispersion A»

The following components were dissolved for one hour while heated at 70°C, and thus an aqueous phase composition A was obtained.

- Sucrose stearic acid ester (HLB = 16)    33.0 g
- Decaglyceryl monooleate (HLB = 12)    67.0 g
- Glycerin    450.0 g
- Pure water    300.0 g

The following components were dissolved for one hour while heated at 70°C, and thus an oil phase composition A was obtained.

- Haematococcus alga extract
(percentage content of astaxanthins 20% by mass)    15.0 g
- Mixed tocopherols
(RIKEN E OIL 800, manufactured by Riken Vitamin Co., Ltd.)    32.0 g
- Medium-chain fatty acid glycerides
(manufactured by Kao Corp., COCONARD MCT)    93.0 g
- Lecithin (SLP paste, manufactured by Tsuji Oil Mills Co., Ltd., derived from soybean) 10.0 g

The aqueous phase composition A obtained as described above was stirred (10,000 rpm) in a homogenizer (model name: HP93, manufactured by SMT Co., Ltd.) while the temperature was maintained at 70°C, and the oil phase composition A was added to the aqueous phase composition A to obtain a preliminary dispersion. Subsequently, the preliminary dispersion thus obtained was cooled to about 40°C, and high pressure emulsification was performed at a pressure of 200 MPa using an ULTIMIZER HJP-25005 (manufactured by Sugino Machine, Ltd.). Thereafter, the resultant was filtered through a microfilter having an average pore diameter of 1 $\mu$m, and thus an astaxanthin dispersion A (percentage content of astaxanthin: 0.3% by mass) was produced.

The astaxanthin dispersion A thus obtained was diluted with MilliQ water to 1% by mass, and the particle diameter of the dispersed particles was measured using a particle size analyzer FPAR-1000 (Otsuka Electronics Co., Ltd). The particle diameter was 58 nm (median diameter (d50)).

«Preparation of lycopene dispersion B»

[Oil phase composition B]

- Tomato oleoresin    0.67 g
(manufactured by Sunbright Co., Ltd., LYC-O-MATO 15%, containing 15% of lycopene)
- Lecithin (manufactured by Riken Vitamin Co., Ltd., LECION P, derived from soybean)    1.0 g
- Medium-chain fatty acid glycerides (manufactured by Kao Corp., COCONARD MT)    10.7 g

[Aqueous phase composition B]

- Decaglyceryl oleate-10　　　8.0 g
(manufactured by Nikko Chemicals Co., Ltd., DECAGLYN 1-O)
- Sucrose stearic acid ester　　2.0 g
(manufactured by Mitsubishi Kagaku Foods Corp., RYOTO SUGAR ESTER S-1670)
- Glycerin　　　45.0 g
- Purified water　　　30.0 g

The various components used in the aqueous phase composition B were weighed in a container according to the composition described above, and the components were heated and mixed under stirring in a constant temperature tank at 70°C. It was confirmed that the components were thoroughly mixed, and the mixture was maintained at 70°C. Thus, an aqueous phase composition B was obtained.

Furthermore, the various components used in the oil phase composition B were weighed in a container according to the composition described above, and the components were heated and mixed for 5 minutes under stirring on a hot plate at 150°C. It was confirmed that the components were thoroughly mixed, and thus an oil phase composition B was obtained.

The aqueous phase composition B thus obtained was added to the oil phase composition B, and the mixture was mixed under stirring and was dispersed using an ultrasonic homogenizer. Subsequently, a crude dispersion thus obtained was further subjected to high pressure emulsification at 200 MPa using an ultrahigh pressure emulsifying apparatus (ULTIMIZER, manufactured by Sugino Machine, Ltd.). Thus, a lycopene dispersion B (percentage content of lycopene: 0.1% by mass) was produced.

The lycopene dispersion thus obtained was diluted with MilliQ water to 1% by mass, and the particle diameter of the dispersed particles was measured using a concentrated system particle size analyzer FPAR-1000 (Otsuka Electronics Co., Ltd.). The particle diameter was 52 nm (median diameter (d50)).

«Tomato extract, krill extract, and Haematococcus alga extract»

The details of the tomato extract, krill extract, and Haematococcus alga extract used in the production of the various topical skin preparations of Examples and Comparative Examples are as follows.

- Tomato extract (percentage content of lycopene: 0.1% by mass, product name: LYC-O-MATO 6% [manufactured by Sunbright Co., Ltd.] was diluted with glycerin)
- Krill extract (percentage content of astaxanthin: 0.6% by mass)
- Haematococcus alga extract (percentage content of astaxanthin: 0.3% by mass, product name: ASTOTS-S [manufactured by Takedashiki Co., Ltd.] was diluted with glycerin)

<Production of topical skin preparation>

Topical skin preparations (skin toner or essence) having the compositions indicated in the following Table 3 were produced as appropriate. Meanwhile, all of Examples 2-1 to 2-8 and Comparative Example 2-1 were topical skin preparations having transparency. Furthermore, regarding magnesium ascorbyl phosphate used in the formulations, a C-MATE manufactured by BASF Japan, Ltd., a NIKKOL VC-PMG manufactured by Nikko Chemicals Co., Ltd., or the like can be used.

<Evaluation of stability over time>

Evaluation (1): Measurement of absorbance change ratio

For the various topical skin preparations of Examples and Comparative Examples immediately after production, and after the passage of a time period of two weeks at 40°C, the absorbance for light having a wavelength of 625 nm was measured using a spectrophotometer (V-630iRM) manufactured by JASCO, Ltd. Using the measurement results thus obtained, the change ratio (%) of absorbance was calculated by the following formula. The results are presented in Table 3.

$$\text{Change ratio (\%)} = \text{Absorbance after the passage of a time period of two weeks at}$$
$$40°C/\text{absorbance immediately after production} \times 100$$

A smaller change ratio (%) implies excellent stability over time.

Evaluation (2): Evaluation of preparation stability

For the various topical skin preparations of Examples and Comparative Examples immediately after production, and after the passage of a time period of two weeks at 40°C, the absorbance for light having a wavelength of 625 nm was measured using a spectrophotometer (V-630iRM) manufactured by JASCO, Ltd. Using the measurement results thus obtained, the change ratio (%) of absorbance was calculated by the following formula. Using the results thus obtained, the preparation stability was evaluated on the basis of the evaluation criteria described below. The results are presented in Table 3.

$$\text{Change ratio (\%)} = \text{Absorbance after the passage of a time period of two weeks at}$$
$$40°C/\text{absorbance immediately after production} \times 100$$

A smaller change ratio (%) implies excellent stability over time.

- Evaluation criteria -

S: 175% or less
A: More than 175% to 300% or less
B: More than 300%

Evaluation (3): Evaluation of transparency after passage of time

For the various topical skin preparations of Examples and Comparative Examples immediately after the passage of a time period of two weeks at 40°C, the absorbance for light having a wavelength of 625 nm was measured using a spectrophotometer (V-630iRM) manufactured by JASCO, Ltd. Using the measurement results thus obtained, transparency after the passage of time was evaluated on the basis of the evaluation criteria described below. The results are presented in Table 3.

- Evaluation criteria -

A: Absorbance is 0.1 or less.
B: Absorbance is more than 0.1.

[Table 3]

| | | Example 2-1 | Example 2-2 | Example 2-3 | Example 2-4 | Example 2-5 | Example 2-6 | Example 2-7 | Example 2-8 | Comparative Example 2-1 |
|---|---|---|---|---|---|---|---|---|---|---|
| | | (skin toner) | (essence) | (skin toner) | (skin toner) | (skin toner) | (essence) | (skin toner) | (skin toner) | (skin toner) |
| Composition | Haematococcus alga extract (containing 0.3 mass% of astaxanthin) | | | 0.05 | | | | | | |
| | Astaxanthin dispersion A (containing 0.3 mass% of astaxanthin) | | | | 0.05 | 0.5 | 1 | 1 | 5 | 15 |
| | Krill extract (containing 0.6 mass% of astaxanthin) | 0.05 | 0.3 | | | | | | | 30 |
| | Tomato extract (containing 0.1 mass% of lycopene) | 2 | 0.3 | | | | | | | |
| | Lycopene dispersion B (containing 0.1 mass% of lycopene) | | | 0.04 | 0.1 | 0.1 | 0.1 | 5 | 4 | 15 |
| | Glycerin | 5 | 5 | 5 | 5 | 5 | 5 | 5 | 5 | 5 |
| | 1,3-Butylene glycol | 6.5 | 6.5 | 6.5 | 6.5 | 6.5 | 6.5 | 6.5 | 6.5 | 6.5 |
| | Polyoxyethylene sorbitan monolauric acid ester | 1.2 | 1.2 | 1.2 | 1.2 | 1.2 | 1.2 | 1.2 | 1.2 | 1.2 |
| | Ethanol | 12 | 6 | 12 | 12 | 12 | 12 | 12 | 12 | 12 |
| | Magnesium ascorbyl phosphate | 0.5 | 0.2 | 0.5 | 0.5 | 0.5 | 0.5 | 0.5 | 0.5 | 0.5 |
| | Xanthan gum | 0.2 | 0.1 | 0.2 | 0.2 | 0.2 | 0.1 | 0.2 | 0.2 | 0.2 |
| | Ceramides | | | | | | 0.1 | | | |
| | Carboxyvinyl polymer | | 0.1 | | | | | | | |
| | Decaglyceryl monooleate | 0.05 | 0.2 | 0.2 | | | | | | |
| | Sucrose stearic acid ester | | 0.1 | 0.5 | 0.2 | | | | | |

(Total amount: 100 % by mass)

| | | Example 2-1 (skin toner) | Example 2-2 (essence) | Example 2-3 (skin toner) | Example 2-4 (skin toner) | Example 2-5 (skin toner) | Example 2-6 (essence) | Example 2-7 (skin toner) | Example 2-8 (skin toner) | Comparative Example 2-1 (skin toner) |
|---|---|---|---|---|---|---|---|---|---|---|
| | | | | | | | | | | (Total amount: 100 % by mass) |
| | Citric acid | 0.05 | 0.05 | | 0.05 | 0.05 | 0.05 | 0.05 | 0.05 | 0.05 |
| | Sodium citrate | | 0.1 | | 0.1 | 0.1 | 0.1 | 0.1 | 0.1 | 0.1 |
| | Collagen | 1 | 1 | 1 | 1 | 1 | 1 | 1 | 1 | 1 |
| | N-acetylhydroxyproline | 1 | 1 | 1 | 1 | 1 | 1 | 1 | 1 | 1 |
| | Methyl para-oxybenzoate | 0.02 | 0.02 | 0.02 | 0.02 | 0.02 | 0.02 | 0.02 | 0.02 | 0.02 |
| | Rose extract oil | | | | | | 0.001 | | | |
| | Rose extract water | | 0.001 | | | | | | | |
| | Fragrance | Adequate amount | Adequate amount | Adequate amount | Adequate amount | Adequate amount | Adequate amount | Adequate amount | Adequate amount | Adequate amount |
| | Polyoxyethylene hardened castor oil | 0.2 | 0.2 | 0.2 | 0.2 | 0.2 | 0.2 | 0.2 | 0.2 | 0.2 |
| | Purified water | Balance | Balance | Balance | Balance | Balance | Balance | Balance | Balance | Balance |
| Lycopene content (mass%) | | 0.0020 | 0.0003 | 0.00004 | 0.0001 | 0.0001 | 0.0001 | 0.005 | 0.004 | 0.015 |
| Astaxanthin content (mass%) | | 0.0003 | 0.0018 | 0.00015 | 0.00015 | 0.0015 | 0.003 | 0.003 | 0.015 | 0.225 |
| Total content (mass%) | | 0.0023 | 0.0021 | 0.00019 | 0.00025 | 0.0016 | 0.0031 | 0.008 | 0.019 | 0.24 |
| Lycopene/astaxanthin mass ratio | | 6.67 | 0.17 | 0.27 | 0.67 | 0.07 | 0.03 | 1.67 | 0.27 | 0.07 |
| Tocopherols content (mass%) | | - | - | - | 0.0016 | 0.016 | 0.032 | 0.032 | 0.160 | 0.480 |
| Tocopherols/lycopene + astaxanthin mass ratio | | - | - | - | 6.4 | 10.0 | 10.3 | 4.0 | 8.4 | 2.0 |

EP 2 818 154 A1

(continued)

|  |  | Example 2-1 | Example 2-2 | Example 2-3 | Example 2-4 | Example 2-5 | Example 2-6 | Example 2-7 | Example 2-8 | Comparative Example 2-1 |
|---|---|---|---|---|---|---|---|---|---|---|
|  |  | (Total amount: 100 % by mass) | | | | | | | | |
|  |  | (skin toner) | (essence) | (skin toner) | (skin toner) | (skin toner) | (essence) | (skin toner) | (skin toner) | (skin toner) |
| Stability evaluation | (1): Absorbance immediately after production (at 625 nm) | 0.021 | 0.012 | 0.003 | 0.004 | 0.013 | 0.015 | 0.034 | 0.054 | 0.521 |
|  | (1): Absorbance after two weeks at 40°C (at 625 nm) | 0.039 | 0.029 | 0.008 | 0.005 | 0.021 | 0.025 | 0.042 | 0.082 | 1.65 |
|  | (1): Change ratio | 186% | 242% | 267% | 125% | 162% | 167% | 124% | 152% | 317% |
|  | (2): Preparation stability | A | A | A | S | S | S | S | S | B |
|  | (3): Transparency after passage of time | A | A | A | A | A | A | A | A | B |

In Table 3, tocopherols refers to a generic term for tocopherol and derivatives thereof.

Furthermore, the tocopherols included in the various topical skin preparations of Examples 2-3 to 2-8 and Comparative Example 2-1 were derived from the mixed tocopherols used in the production of the astaxanthin dispersion A.

As shown in Table 3, it can be seen that all of the various topical skin preparations of the Examples that include lycopene and astaxanthin with the total content according to the invention have excellent stability when formulated into preparations.

Furthermore, all of the various topical skin preparations of Examples 2-5 to 2-8 are examples that used fatty acid esters of monosaccharides or polysaccharides, polyglycerin fatty acid esters, and decaglyceryl monooleate, sucrose stearic acid ester, and soybean lecithin as phospholipids, which are incorporated components suitable for the invention. As shown in Table 3, it can be seen that when these various components are incorporated, topical skin preparations having excellent stability when formulated into preparations can be obtained.

[Example 2-9]

A milky lotion having the following composition was produced (total amount: 100 % by mass).

| (Component) | (mass%) |
|---|---|
| • Tomato extract (containing 0.1 mass% of lycopene) | 0.1 |
| • Lycopene dispersion (containing 0.1 mass% of lycopene) | 0.1 |
| • Lutein | 0.01 |
| • Haematococcus alga extract (containing 0.3 mass% of astaxanthin) | 0.5 |
| • Astaxanthin dispersion (containing 0.3 mass% of astaxanthin) | 0.5 |
| • Squalane | 8.0 |
| • Jojoba oil | 7.0 |
| • Glyceryl para-aminobenzoate | 1.0 |
| • Cetyl alcohol | 1.5 |
| • Glycerin monostearate | 2.0 |
| • Polyoxyethylene cetyl ether | 3.0 |
| • Polyoxyethylene sorbitan monooleate | 2.0 |
| • 1,3-Butylene glycol | 1.0 |
| • Glycerin | 2.0 |
| • Methylparaben | 0.04 |
| • Sucrose stearate | 0.1 |
| • Polyglyceryl-10 oleate | 0.1 |
| • Tocopherol acetate | 0.01 |
| • Phenoxyethanol | 0.2 |
| • Collagen | 1.0 |
| • Sodium citrate | 1.0 |
| • Fragrance | Adequate amount |
| • Purified water | Balance |

With respect to the total mass of the milky lotion having the above composition, the lycopene content is 0.0002% by mass, the astaxanthin content is 0.003% by mass, and the tocopherols content is 0.026% by mass.

The mass ratio of lycopene and astaxanthin, and the mass ratio of tocopherols and lycopene with astaxanthin are as follows.

$$\text{Lycopene/astaxanthin} = 0.67$$

$$\text{Tocopherols/(lycopene + astaxanthin)} = 8.1$$

(here, "tocopherols" refers to a generic term for tocopherol and derivatives thereof.)

Meanwhile, it was confirmed that in regard to the above composition, a milky lotion can be similarly produced even when fucoxanthin is used instead of lutein.

[Example 2-10]

A cleansing oil having the following composition was produced (total amount: 100% by mass).

| (Component) | (mass%) |
|---|---|
| • Ethylhexyl palmitate | 70.0 |
| • Tomato extract (containing 0.1 mass% of lycopene) | 0.3 |
| • Haematococcus alga extract (containing 0.3 mass% of astaxanthin) | 0.2 |
| • Tocopherol | 0.7 |
| • Damask rose oil | Trace amount |
| • Phenoxyethanol | 0.25 |
| • Diisostearyl malate | 5.0 |
| • Polyglyceryl-10 trilaurate | 15.0 |
| • Hydrogenated polyisobutene | Balance |

With respect to the total mass of the cleansing oil having the above composition, the lycopene content is 0.003% by mass, the astaxanthin content is 0.006% by mass, and the tocopherol content is 0.4% by mass.
The mass ratio between lycopene and astaxanthin, and the mass ratio between tocopherols and lycopene with astaxanthin are as follows.

$$\text{Lycopene/astaxanthin} = 0.5$$

$$\text{Tocopherols/(lycopene + astaxanthin)} = 44.4$$

(here, "tocopherols" refers to a generic term for tocopherol and derivatives thereof.)

[Example 2-11]

A cleansing foam having the following composition was produced (total amount: 100% by mass).

| (Component) | (mass%) |
|---|---|
| • Lycopene dispersion (containing 0.1 mass% of lycopene) | 0.4 |
| • Myristic acid | 25.0 |
| • Stearic acid | 8.0 |
| • Glyceryl stearate | 5.0 |
| • Sodium lauroyl glutamate | 1.0 |
| • Phenoxyethanol | 0.25 |
| • Polyquaternium-39 | 1.0 |
| • Water-soluble collagen | 1.0 |
| • Potassium hydroxide | 7.0 |
| • Tomato extract (containing 0.1 mass% of lycopene) | 0.1 |
| • Haematococcus alga extract (containing 0.3 mass% of astaxanthin) | 0.1 |
| • Tocopherol | 0.15 |
| • Purified water | Balance |

With respect to the total mass of the cleansing foam having the above composition, the lycopene content is 0.001% by mass, the astaxanthin content is 0.003% by mass, and the tocopherols content is 0.15% by mass.
The mass ratio between lycopene and astaxanthin, and the mass ratio between tocopherols and lycopene with astaxanthin are as follows.

$$\text{Lycopene/astaxanthin} = 0.33$$

$$\text{Tocopherols/(lycopene + astaxanthin)} = 37.5$$

(here, "tocopherols" refers to a generic term for tocopherol and derivatives thereof.)

[Example 2-12]

A cream having the following composition was produced (total amount: 100% by mass).
Furthermore, regarding magnesium ascorbyl phosphate used in the formulations, a C-MATE manufactured by BASF Japan, Ltd., a NIKKOL VC-PMG manufactured by Nikko Chemicals Co., Ltd., or the like can be used.

| (Component) | (mass%) |
|---|---|
| • Lycopene dispersion (containing 0.1 mass% of lycopene) | 0.4 |
| • Astaxanthin dispersion (containing 0.3 mass% of astaxanthin) | 0.2 |
| • Cetostearyl alcohol | 3.0 |
| • Glycerin fatty acid ester | 2.0 |
| • Polyoxyethylene (20) sorbitan monooleate | 1.0 |
| • Sorbitan monostearate | 1.0 |
| • N-stearoyl-N-methyltaurine sodium | 0.5 |
| • Petrolatum | 5.0 |
| • Lecithin | 0.5 |
| • Dimethylpolysiloxane (100 mPa·s) | 3.0 |
| • Glyceryl tri-2-ethylhexanoate | 20.0 |
| • Lactic acid | 1.0 |
| • Magnesium ascorbate phosphate | 0.5 |
| • Dipropylene glycol | 10.0 |
| • Sodium citrate | 0.5 |
| • Titanium oxide | 0.1 |
| • Fragrance | Adequate amount |
| • Disodium edetate | 0.03 |
| • Ethyl para-oxybenzoate | 0.05 |
| • Purified water | Balance |

With respect to the total mass of the cream having the above composition, the lycopene content is 0.004% by mass, the astaxanthin content is 0.006% by mass, and the tocopherols content is 0.064% by mass.
The mass ratio between lycopene and astaxanthin, and the mass ratio between tocopherols and lycopene with astaxanthin are as follows.

$$\text{Lycopene/astaxanthin} = 0.67$$

$$\text{Tocopherols/(lycopene + astaxanthin)} = 6.4$$

(here, "tocopherols" refers to a generic term for tocopherol and derivatives thereof.)

<Test for verifying anti-wrinkle effect in human beings>

A test for verifying an anti-wrinkle effect in human beings was carried out using the cream of Example 2-12 produced as described above, and a comparative dispersion (cream) produced by replacing the tomato extract and Haematococcus alga extract used in the production of the foregoing cream with petrolatum.
The cream of Example 2-12 and the comparative dispersion were respectively used around the eyes of ten subjects of ages of from 35 to 60 for 4 weeks (number of applications: 2 times/day), and changes in the wrinkles before and after the use were checked.
Checking of the changes in the wrinkles was carried out by measuring wrinkle scores using an image analyzer VISIA (manufactured by Integral Corp). A lower score implies fewer wrinkles.

From the scores thus obtained, a wrinkle improvement ratio (%) was calculated based on the following formula.

$$\text{Wrinkle improvement ratio (\%)} = \text{Score after use/score before use} \times 100$$

The results are presented in Table 4.

[Table 4]

|  | Example 2-12 | Comparative emulsion |
|---|---|---|
| Score before use | 1.05 | 0.9 |
| Score after use | 0.76 | 0.82 |
| Wrinkle improvement ratio | 28% | 9% |

As shown in Table 4, it was confirmed that the cream of the Example containing lycopene and astaxanthin with the total content according to the invention has an anti-wrinkle effect as compared with the comparative dispersion.

[Example 2-13]

High pressure emulsification was carried out in the same manner as in the case of the lycopene dispersion used in Example 2-1, except that 0.6 g of tocopherol was added to the oil phase composition B obtained in the production of the lycopene dispersion B, and adjustment by adding water was carried out using the same purified water as that used in the aqueous composition B, to make up a total amount of 100 g. Thus, a lycopene dispersion C was produced.

The lycopene dispersion C thus obtained was diluted with MilliQ water to 1% by mass, and the particle diameter of the dispersed particles was measured using a concentrated system particle size analyzer FPAR-1000 (Otsuka Electronics Co., Ltd). The particle diameter was 58 nm (median diameter (d50)).

A stable cosmetic material (essence) could be obtained even when the tomato extract used in Example 2-2 was replaced with 0.3 g of the lycopene dispersion C.

The entire disclosure of Japanese Patent Application No. 2012-039374, filed February 24, 2012, is incorporated herein by reference.

All publications, patent applications, and technical standards mentioned in this specification are herein incorporated by reference to the same extent as if each individual publication, patent application, or technical standard was specifically and individually indicated to be incorporated by reference.

**Claims**

1.    A topical skin preparation, comprising: lycopene and astaxanthin, a total content of the lycopene and the astaxanthin being in a range of from 0.00001% by mass to 0.2% by mass.

2.    The topical skin preparation according to claim 1, wherein the lycopene is incorporated as a dispersion containing lycopene.

3.    The topical skin preparation according to claim 2, comprising the dispersion that contains the lycopene; at least one selected from a fatty acid ester of a monosaccharide, a fatty acid ester of a polysaccharide, a polyglycerin fatty acid ester of a monosaccharide, or a polyglycerin fatty acid ester of a polysaccharide; and a lecithin.

4.    The topical skin preparation according to any one of claims 1 to 3, wherein the astaxanthin is incorporated as a dispersion containing astaxanthin.

5.    The topical skin preparation according to claim 4, comprising the dispersion that contains the astaxanthin; at least one selected from a fatty acid ester of a monosaccharide, a fatty acid ester of a polysaccharide, a polyglycerin fatty acid ester of a monosaccharide, or a polyglycerin fatty acid ester of a polysaccharide; and a lecithin.

6.    The topical skin preparation according to any one of claims 1 to 5, wherein a mass ratio of the lycopene and the astaxanthin is from 0.01 : 1 to 25 : 1.

7. The topical skin preparation according to any one of claims 1 to 6, further comprising at least one selected from the compound group consisting of tocopherol and derivatives thereof.

8. The topical skin preparation according to claim 7, wherein a mass ratio between the lycopene with the astaxanthin, and the at least one selected from the compound group consisting of tocopherol and derivatives thereof, is from 1 : 50 to 1 : 0.5.

9. The topical skin preparation according to any one of claims 1 to 8, which is an aqueous cosmetic.

10. A normal skin cell activating agent, comprising: lycopene and astaxanthin, a total content of the lycopene and the astaxanthin being in a range of from 0.00001% by mass to 0.2% by mass.

11. The normal skin cell activating agent according to claim 10, wherein a mass ratio between the lycopene and the astaxanthin is from 0.01 : 1 to 25 : 1.

12. A topical skin preparation comprising the normal skin cell activating agent according to claim 10 or 11.

13. A cosmetic comprising the normal skin cell activating agent according to claim 10 or 11.

**EP 2 818 154 A1**

## INTERNATIONAL SEARCH REPORT

| | International application No. |
|---|---|
| | PCT/JP2013/054611 |

**A. CLASSIFICATION OF SUBJECT MATTER**
See extra sheet.

According to International Patent Classification (IPC) or to both national classification and IPC

**B. FIELDS SEARCHED**

Minimum documentation searched (classification system followed by classification symbols)
A61K8/31, A61K8/35, A61K8/37, A61K8/55, A61K8/60, A61K8/67, A61K31/01, A61K31/122, A61K47/24, A61K47/26, A61K47/34, A61K47/36, A61P17/00, A61P43/00, A61Q1/14, A61Q19/00, A61Q19/08, A61Q19/10

Documentation searched other than minimum documentation to the extent that such documents are included in the fields searched
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2007-160287 A (ITO Co., Ltd.), 28 June 2007 (28.06.2007), example 4; paragraphs [0077] to [0080] & WO 2007/066832 A1 | 1,2,4,6-13 |
| X | JP 2005-343880 A (ITO Co., Ltd.), 15 December 2005 (15.12.2005), examples 3 to 13; paragraphs [0071] to [0086] (Family: none) | 1,2,4,6-13 |
| X | JP 2011-241176 A (Fujifilm Corp.), 01 December 2011 (01.12.2011), claims; paragraphs [0011] to [0014], [0016] to [0027], [0098]; example 2 (Family: none) | 1-13 |

☒ Further documents are listed in the continuation of Box C.　　☐ See patent family annex.

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 May, 2013 (13.05.13) | 21 May, 2013 (21.05.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2013/054611 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2008-100991 A (Rohto Pharmaceutical Co., Ltd.), 01 May 2008 (01.05.2008), claims; paragraphs [0009], [0010]; examples 1, 5, 6 (Family: none) | 1,2,4,6-13 |
| Y | Taeko IINO et al., "LDL Hisankasei o Shihyo to shita Astaxanthin to Lycopene no Sogo Sayo", The Japanese Society of Nutrition and Food science Taikai Koen Yoshishu, 2001.04, 55th, page 74, 2G-07a | 1-13 |
| Y | Yasuyuki IZUMI et al., "Development of the dietary supplements, 'Astalift supplement' and 'Astalift drink'", Fuji Film research & development, 25 March 2009 (25.03.2009), no.54, pages 21 to 24, page 22, left column, lines 18 to 26, left column, line 47 to right column, line 1, table 1 | 1-13 |
| Y | Masao SHIMURA, "21 Seiki, Wadai no Shokuhin Sozai, Astaxanthin o Ou", Food Research, 01 April 2002 (01.04.2002), vol.562, pages 58 to 60 | 1-13 |
| Y | JP 2008-013751 A (Fujifilm Corp.), 24 January 2008 (24.01.2008), claims; paragraphs [0003], [0007], [0014]; examples & US 2007/0286930 A1 & EP 1864578 A1 | 1-13 |
| P,X | WO 2013/002278 A1 (Fujifilm Corp.), 03 January 2013 (03.01.2013), claims; examples (Family: none) | 1-13 |
| P,A | Toshihiko SAWADA et al., "Astaxanthin-Lycopene Kongokei no Kosankano", The 92nd Annual Meeting of the Chemical Society of Japan in Spring (2012) Koen Yokoshu III, 09 March 2012 (09.03.2012), page 923, 3G3-15 | 1-13 |
| P,A | Eriko INUI, "Nano Lycopene o Haigo shita Antiageing Skin Care Shohin no Kaihatsu", Fragrance Journal, 15 February 2013 (15.02.2013), vol.41, no.2, pages 34 to 41 | 1-13 |

Form PCT/ISA/210 (continuation of second sheet) (July 2009)

**INTERNATIONAL SEARCH REPORT**

| International application No. |
| --- |
| PCT/JP2013/054611 |

Continuation of A. CLASSIFICATION OF SUBJECT MATTER
  (International Patent Classification (IPC))

A61K8/31(2006.01)i, A61K8/35(2006.01)i, A61K8/37(2006.01)i,
A61K8/55(2006.01)i, A61K8/60(2006.01)i, A61K8/67(2006.01)i,
A61K31/01(2006.01)i, A61K31/122(2006.01)i, A61K47/24(2006.01)i,
A61K47/26(2006.01)i, A61K47/34(2006.01)i, A61K47/36(2006.01)i,
A61P17/00(2006.01)i, A61P43/00(2006.01)i, A61Q1/14(2006.01)i,
A61Q19/00(2006.01)i, A61Q19/08(2006.01)i, A61Q19/10(2006.01)i

(According to International Patent Classification (IPC) or to both national classification and IPC)

Form PCT/ISA/210 (extra sheet) (July 2009)

# EP 2 818 154 A1

**Patent documents cited in the description**

- JP 2009511570 A
- JP 2000229827 A
- JP 2002128651 A
- JP 3507055 B
- JP 3389580 B
- JP H0568585 A
- JP 2012039374 A

**Non-patent literature cited in the description**

- *Mol. Cancer Ther.,* 2005, vol. 4 (1), 177-186